# EUROPÄISCHE PATENTANMELDUNG

(11) **EP 1 157 716 A1**
(43) Veröffentlichungstag der Anmeldung: **28.11.2001**
(21) Anmeldenummer: 01111367.7
(22) Anmeldetag: 09.05.2001
(51) Int. Cl.: A61M 25/00

(54) **Katheteranordnung**

(30) Priorität: 22.05.2000 DE 10025266
(71) Anmelder: Jomed GmbH, 72414 Rangendingen (DE)
(72) Erfinder: Schwager, Michael, 8404 Winterthur (CH); Berger, Erwin, 9507 Stettfurt (CH)
(74) Vertreter: Weber, Joachim, Dr.

(57) **Zusammenfassung**

Die Erfindung betrifft eine Katheteranordnung (1) mit einem Führungsdrahttubus (3) zur Aufnahme eines Führungsdrahtes und vorzugsweise einen am Führungsdrahttubus (3) befestigten, expandierbaren Ballon (7), wobei der Führungsdrahttubus (3) eine über das distale Ende (4) des Ballons (7) hinausstehende gebogene Spitze (2) aufweist.

## Beschreibung

Die Erfindung betrifft eine Katheteranordnung gemäß dem Oberbegriff des Anspruches 1.

Eine gattungsgemäße Katheteranordnung, die vorzugsweise einen expandierbaren Ballon aufweist, wird zur Implantation von Stents an Verengungsstellen in Körpergefäßen oder Körperhöhlungen verwendet, um diese Verengungsstellen aufzuweiten und zu stabilisieren.

Zur Führung des Katheters wird ein steuerbarer Führungsdraht verwendet, wobei sich bei der gattungsgemäßen Katheteranordnung Probleme ergeben, wenn der Katheter in eine Gefäßabzweigung eingeführt werden soll. Der Führungsdraht wird hierbei möglichst weit vorgeschoben, um im Bereich der Abzweigung eine steife Führung zu gewährleisten. Eine tiefe Verankerung des Führungsdrahtes in der Abzweigung ist jedoch aus anatomischen Gründen oftmals nicht möglich. Da der Führungsdraht erst mit zunehmendem Abstand von seiner Spitze weg an Steifigkeit zunimmt, kann in vielen Fällen nicht ausgeschlossen werden, dass nur die weiche Führungsdrahtspitze als Führung in der Abzweigung zur Verfügung steht.

Der vorzuschiebende Katheter ist wesentlich steifer als die Führungsdrahtspitze. Somit kann der Führungsdraht keine genügend große Biegekraft auf den Katheter ausüben und wird bei der gattungsgemäßen Katheteranordnung somit häufig aus der Abzweigung herausgedrückt, wenn der steifere Katheter entlang des Führungsdrahtes in Richtung auf die Abzweigung vorgeschoben wird.

Es ist daher Aufgabe der vorliegenden Erfindung, eine Katheteranordnung der im Oberbegriff des Anspruchs 1 angegebenen Art zu schaffen, die ein sicheres Einführen in Gefäßabzweigungen ohne die Gefahr des Herausdrückens des Führungsdrahtes aus der Gefäßabzweigung möglich macht.

Die Lösung dieser Aufgabe erfolgt durch die Merkmale des Anspruches 1.

Bei der erfindungsgemäßen Katheteranordnung weist der Führungsdrahttubus eine über das distale Ende des Ballons hinausstehende gebogene Spitze auf. Dies gewährleistet ein sicheres Einführen in Abzweigungen eines Gefäßsystems, da der zuvor in die Abzweigung vorgeschobene Führungsdraht aufgrund der gebogenen Spitze der Katheteranordnung nicht mehr aus der Abzweigung herausgedrückt wird, sondern sich vielmehr die Katheteranordnung problemlos in die Abzweigung einfädelt.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung eines Ausführungsbeispiels anhand der Zeichnung.

Es zeigen:
- Fig. 1: eine schematisch leicht vereinfachte Schnittdarstellung durch eine erfindungsgemäße Katheteranordnung,
- Fig. 2: und 3 die Funktionsweise der erfindungsgemäßen Katheteranordnung gemäß Fig. 1.

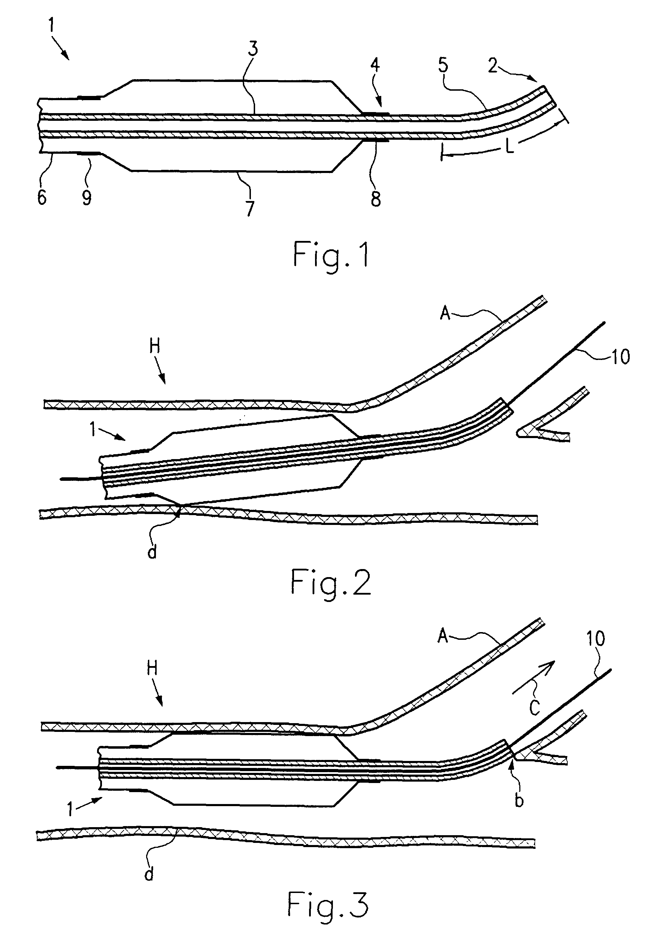

In Fig. 1 ist als ein mögliches Beispiel eine Katheteranordnung 1 zur Implantation eines Stents dargestellt. Die Katheteranordnung 1 weist einen Führungsdrahttubus 3 auf, in dem ein Führungsdraht 10 angeordnet werden kann. Der Führungsdraht und der Führungsdrahttubus 3 bilden gemeinsam eine Führungseinrichtung.

Wie in Fig. 1 gezeigt, umfasst die Katheteranordnung 1 weiter einen Ballon 7 und einen Tubus 6. Der Ballon 7 ist mit seinem distalen Ende über einen distalen Befestigungsbereich 8 am Führungsdrahttubus 3 befestigt und mit seinem proximalen Ende über einen proximalen Befestigungsbereich 9 am Tubus 6 befestigt. Hierbei wird unter "distalem Ende" "zum Herzen hinführend" und unter "proximalem Ende" "vom Herzen wegführend" verstanden. Der mit dem Ballon 7 verbundene Tubus 6 dient dazu, ein geeignetes Medium wie z.B. eine Kochsalzlösung in den hohlen Innenraum des Ballons 7 zuzuführen und diesen an der verengten Stelle zu expandieren. Dadurch kann ein um den Ballon 7 angebrachter Stent (nicht dargestellt) expandiert werden und seinerseits die Aufweitung der verengten Stelle im Körperhohlgefäß als Gefäßstütze stabilisieren.

Die erfindungsgemäße Katheteranordnung 1 weist ferner an ihrem distalen Ende 4 eine gebogene Spitze 2 auf, die eine über das distale Ende 4 des Ballons 7 hinausstehende Verlängerung des Führungsdrahttubus darstellt.

Die gebogene Spitze 2 weist vorzugsweise einen Krümmungsradius von 7 mm auf. Es ist jedoch grundsätzlich auch möglich, andere Krümmungsradien je nach Einsatzzweck der erfindungsgemäßen Katheteranordnung 1 vorzusehen.

Die Länge L des gebogenen Abschnittes 5 der Spitze 2 beträgt je Anwendungsfall mindestens 2 mm und mehr ist jedoch bei einer besonders bevorzugten Ausführungsform größer als 5 mm.

In den Fig. 2 und 3 ist die Funktionsweise der erfindungsgemäßen Katheteranordnung 1 erläutert. Die Fig. 2 und 3 verdeutlichen hierbei schematisch stark vereinfacht ein Hohlgefäß H, in dessen Abzweigung A die Katheteranordnung 1 platziert werden soll. Hierzu wird ein Führungsdraht 10 zunächst in die Abzweigung A vorgeschoben und anschließend die Katheteranordnung 1 über den Führungsdraht 10 in der Abzweigung A platziert. Die Fig. 2 und 3 verdeutlichen hierbei, dass die gebogene Spitze 2 das Vorschieben in die Abzweigung A gewährleistet, da die wesentlich steifere Kätheteranordnung 1 aufgrund der gebogenen Spitze 2 den Führungsdraht 10 nicht mehr aus der Abzweigung A herausdrücken kann, wenn der Führungsdraht 10 nicht tief in dieser Abzweigung A, beispielsweise aus anatomischen Gründen, verankert werden kann. Durch die gebogene Spitze 2 ergibt sich der Vorteil, dass die Katheteranordnung 1 ohne Krafteinwirkung des Führungsdrahtes 10 in die Abzweigung A eingeleitet werden kann. Hierbei trifft die gebogene Spitze 2 gemäß der Darstellung in Fig. 3 typischerweise auf den Punkt b, sodass beim weiteren Vorschieben die Spitze 2 in der Innenwand des Gefäßes in Richtung c weiter vorgleitet.

Wenn die Spitze 2 aus Reibungsgründen nicht auf Anhieb in Richtung c gleitet, wird die Katheteranordnung 1 um den Berührungspunkt b gebogen und berührt bei Punkt d die Innenwand des Gefäßes, was aus Fig. 2 ersichtlich ist. Dies wiederum ergibt eine Reaktionskraft auf die Katheteranordnung 1 in Richtung auf die Abzweigung A, was das Vorschieben in Richtung c, ohne die Gefahr des Herausdrückens des Führungsdrahtes 10 möglich macht.

## Patentansprüche

1. Katheteranordnung (1) mit
- einem Führungsdrahttubus (3) zur Aufnahme eines Führungsdrahts (10) und
- vorzugsweise einem expandierbaren Ballon (7),
**dadurch gekennzeichnet, dass**
der Führungsdrahttubus (3) eine über das distale Ende (4) des Ballons (7) hinausstehende gebogene Spitze (2) aufweist.

2. Katheteranordnung nach Anspruch 1, **dadurch gekennzeichnet, dass** die Spitze (2) einen Krümmungsradius von etwa 7 mm aufweist.

3. Katheteranordnung nach Anspruch 2, **dadurch gekennzeichnet, dass** die Länge (L) des gebogenen Abschnitts (5) der Spitze (2) mindestens 4 mm, vorzugsweise mehr als 5 mm beträgt.
